Europäisches Patentamt

European Patent Office

Office européen des brevets

(19)

(11) Publication number: **0 311 364 B1**

# EUROPEAN PATENT SPECIFICATION

(12)

(45) Date of publication of patent specification: **07.10.92**  (51) Int. Cl.5: **A61L 15/16**

(21) Application number: **88309258.7**

(22) Date of filing: **05.10.88**

(54) Wound dressing with activated carbon.

(30) Priority: **06.10.87 GB 8723447**

(43) Date of publication of application:
**12.04.89 Bulletin  89/15**

(45) Publication of the grant of the patent:
**07.10.92 Bulletin  92/41**

(84) Designated Contracting States:
**AT BE CH DE ES FR GB IT LI NL**

(56) References cited:
EP-A- 0 053 936        EP-A- 0 117 438
GB-A- 386 067          GB-A- 1 301 101
GB-A- 2 127 389        GB-A- 2 164 327

CARBON, vol. 26, no. 1, 1988, page 7-11,
Pergamon Press, Oxford, GB; J.J. FREEMAN
et al.: "Studies of activated charcoal cloth.
III. Mesopore development induced by phos-
phate impregnants"

(73) Proprietor: **JOHNSON & JOHNSON**
**One Johnson & Johnson Plaza**
**New Brunswick, NJ 08933-7003(US)**

(72) Inventor: **Wright, Joanne Evelyn**
**19 Isambard Close**
**Cowley Middlesex, UB8 3RX(GB)**
Inventor: **Freeman, John James**
**23 Clifton Road**
**Southall Middlesex, UB2 5OP(GB)**
Inventor: **Sing, Kenneth Stafford William**
**Oak Grove Coombe Lane Hughenden Valley**
**High Wycombe Buckinghamshire, HP14**
**4NX(GB)**
Inventor: **Jackson, Stuart Windust**
**5 Piece Fields Threshfield**
**NR. Skipton North Yorkshire(GB)**
Inventor: **Smith, Rory James Maxwell**
**High Dene Hebden**
**NR. Skipton North Yorkshire(GB)**

(74) Representative: **Fisher, Adrian John et al**
**CARPMAELS & RANSFORD 43 Bloomsbury**
**Square**
**London WC1A 2RA(GB)**

Rank Xerox (UK) Business Services

## Description

This invention relates to wound dressings, and more particularly to wound dressings which include a layer of mesoporous activated carbon. The invention also relates to a method of making mesoporous activated carbon for use in a wound dressing.

It has long been known that charcoal may be rendered highly adsorptive by heating in a suitable atmosphere, the product of such treatment commonly being referred to as "activated charcoal" or "activated carbon". Many applications have been found for the adsorptive properties of activated carbon, particularly in the adsorption of undesirable contaminants from gases and liquids. Until relatively recently, activated carbon has generally been used in the form of small granules, because the known processes for producing activated carbon resulted in a product which was extremely friable and difficult to handle in any other form.

GB-1301101 discloses a method of preparing a relatively strong and flexible activated carbon cloth from fibrous carbohydrate starting materials. In this method, the starting materials are first pre-treated by the incorporation of certain Lewis acids therein. Such activated carbon cloth has been found to have a particular utility in wound dressings, where it is has been found not only to adsorb noxious odours from infected wounds, but also to adsorb bacteria and thereby promote wound healing.

When fibrous cellulosic materials are pyrolysed by the process disclosed in GB-1301101, the cellulosic material first forms a carbonaceous char, and continued pyrolysis then causes this char to become activated. During the latter activation stage, pores are formed in the char with the result that its specific surface area and its adsorptive capacity are very greatly increased. Generally speaking, the process disclosed in GB-1301101 results in fibrous activated carbon having pores in the micropore range (0 to 2 nm width), even with prolonged pyrolysis.

GB-A-2164327 discloses a method of preparing a fibrous activated carbon including the steps of carbonising and activating cellulose fibre at temperatures between 200°C and 1000°C in an inert atmosphere, wherein, prior to activation, the fibre is impregnated with an impregnating material comprising, in the form of one or more compounds, boron and at least one alkali metal. This process results in activated carbon having another range of larger pores in addition to those obtained by the process disclosed in GB-1301101. Typically, the process of GB-A-2164327 results in activated carbons having from 15 to 50% of their total pore volume represented by mesopores. Mesopores are defined by the IUPAC as pores in which the capillary condensation with the formation of a meniscus of the liquid condensate can take place. They have an effective width of approximately 2 to 50 nm and their presence in activated carbon may be detected by analysis of the adsorption isotherm of the activated carbon, as disclosed by Sing (Pure and Appl. Chem. 57, pp. 603 - 619, 1985).

We have now discovered that mesoporous activated carbon has a property which renders it particularly suitable for use in wound dressings. Although even the mesopores are not large enough to accommodate bacteria, we have found that mesoporous activated carbon adsorbs large molecules such as proteins. In particular, and quite remarkably, it adsorbs bacterial toxins, even in the presence of an enormous excess of blood serum proteins, such as might be expected to be present in wound exudate.

According to the present invention there is provided a wound dressing comprising a layer of activated carbon, wherein at least 10% of the pore volume of the activated carbon is represented by mesopores. Preferably, the mesopore content is from 25 to 90% of the total pore volume, and more especially from 30 to 85%.

Preferably, the activated carbon is in the form of a fibrous web. Fibrous activated carbon may be formed from a natural cellulose (such as cotton), or a regenerated cellulose (such as viscose rayon and cuprammonium rayon). The cellulose diameter will generally be from 2 to 100 microns, and preferably from 5 to 20 microns. The activated carbon fibre will generally be present in the wound dressing of the invention in the form of a felt or non-woven or woven cloth or fabric.

The activated carbon material may be prepared by the method disclosed in GB-A-2164327. Alternatively, however, the activated carbon is prepared by impregnating a cellulosic fibrous material with a liquid medium containing phosphate, an alkali metal and at least one Lewis acid, and then carbonising and activating the impregnated fibrous material by heating in a suitable atmosphere. Activated carbon produced by this method has several advantages over mesoporous carbon cloth produced by the method of GB-A-2164327, including higher mesopore volume and greater physical strength. This combination of properties is surprising in view of the fact that a higher degree of mesoporosity is usually associated with lower physical strength.

The liquid medium which is used in this preferred method is preferably an aqueous solution. The phosphate may conveniently be provided in the form of phosphoric acid, or as a phosphate salt, a hydrogen diphosphate salt, or a dihydrogen phosphate salt of a suitable metal, e.g. the alkali metal. The impregnating solution preferably contains from 1 to 10% w/v of an alkali metal salt and from 1 to 10% w/v by weight of phosphoric acid or phosphate salt, and more preferably from 1.5 to 6% w/v e.g. from 2 to 4% w/v.

The amount of phosphorus impregnated on to the fibre is preferably from 0.01% to 10% by weight, and more preferably from 0.1 to 5%, e.g. from 0.5 to 2% by weight.

When the Lewis acid, which is a halide of zinc, aluminium, calcium, magnesium or iron is incorporated from solution, the solution will normally have a concentration of from 1 to 30% w/v of the Lewis acid, and preferably from 2 to 10% w/v. It is particularly preferred that a mixture of Lewis acids be used, the total concentration of which is from 2 to 10% w/v. For example, the impregnating solution may contain a mixture of aluminium chloride and zinc chloride.

Optionally, other salts, such as ammonium halides and alkali metal halides, may be included, preferably in amounts of from 0 to 5% w/v.

It has been found that particularly desirable results are obtained when the impregnating solution contains a mixture of a phosphate, aluminium chloride, zinc chloride and ammonium chloride, each present at a concentration of from 1.5 to 6% w/v, and more preferably from 2 to 4% w/v.

After impregnation, the fibrous carbohydrate material is dried and then preferably flexed to restore at least most of any flexibility lost by contact with the Lewis acid preparation.

The fibre is preferably carbonised at a temperature between 200°C and 600°C in a vacuum or in an atmosphere which is unreactive at the temperature employed. A suitable atmosphere will usually contain relatively inert gases such as nitrogen, carbon dioxide, carbon monoxide, argon, hydrogen, combustion gases from hydrocarbon fuels, steam or mixtures of two or more of these gases.

Activation of the carbonised fibre is achieved by heating at a temperature between 450°C and 1000°C and preferably between 600°C and 850°C in the presence of a stream of activating gas until the desired porosity (activity) is obtained. The activating gas is preferably steam or carbon dioxide. Activation is preferably carried out for from 1 to 200, more preferably from 15 to 150, and most preferably from 60 to 90 minutes.

In a particularly preferred embodiment of the present invention, the activated carbon material is partially impregnated with an antimicrobial agent, such as silver. Preferably, the activated carbon is impregnated with silver by incorporating a silver salt in the impregnation solution prior to the carbonisation step. For example, silver may be added as silver nitrate, with a complexing agent such as ammonia or sodium thiosulphate also being added to prevent precipitation of insoluble silver salts.

The wound dressing according to the present invention may conveniently contain the activated carbon layer between two facing layers of woven or non-woven fabric. The two facing layers may be the same or different, but the layer which in use contacts the wound is preferably non-adherent to wounds. Spun-bonded nylon webs having a weight of between 10 and 50 gm$^{-2}$ are particularly suitable as facing layers, and such webs having a weight of from 20 to 40 gm$^{-2}$ (e.g. 25 gm$^{-2}$) are especially preferred.

The dressing is sterilised (e.g. by nitrous oxide or radiation) and contained in a bacteria-proof package.

The present invention is further illustrated by the following examples.

Example 1

A sample of viscose rayon cloth was impregnated by immersion for 0.5 minutes in an aqueous solution having the following composition:

sodium dihydrogen phosphate 3% w/v
aluminium chloride 3% w/v
ammonium chloride 3% w/v
zinc chloride 3% 3/v

Excess impregnating solution was then removed by means of blotting paper and the fabric was dried in an oven at 55°C. The sample was then carbonised by heating in an atmosphere of nitrogen with ramp rates of 10°C min$^{-1}$ from room temperature to 600°C and 2°C min$^{-1}$ from 600°C to 800°C. The furnace was then maintained at a temperature of 800°C under an atmosphere of carbon dioxide for 75 minutes to activate the carbonised fabric.

The resulting product was found to have 80% of its pore volume represented by mesopores. Electron microscopy showed these mesopores to be of regular, generally parallel-sided shape.

Example 2

The process of Example 1 was repeated, except that the impregnating solution had the following composition:

phosphoric acid 3% w/v
sodium chloride 1% w/v
aluminium chloride 3% w/v
ammonium chloride 3% w/v
zinc chloride 3% w/v

The resulting product was found to have 30% of its pore volume represented by mesopores, which were of regular, generally parallel-sided shape.

Examples 3 and 4

In these examples, the mesoporous carbon cloth prepared in Example 1 was tested for its ability to adsorb proteins of comparatively low and of medium molecular weights. In place of bacterial toxins, the proteins cytochrome C (MW 12,500) and bovine serum albumin (MW 66,000) were used because they are of similar size to many bacterial toxins but are more readily available and much safer to handle.

Samples of cloth (0.01g) were placed in polystyrene vials of 7 ml capacity and aliquots (1ml) of phosphate buffered saline containing cytochrome C labelled with $^{125}$I were added to each. The samples were gently agitated overnight in an incubator at a temperature of 26.5°C. The samples of cloth were then removed, washed with phosphate buffered saline and the amount of radioactivity present on the samples measured by means of a gamma counter. The specific activity of the original cytochrome C solution was known and therefore the quantity of protein bound to the cloth could be determined and expressed as the number of moles of protein adsorbed per gram of cloth. This procedure was repeated with solutions of cytochrome C having a range of concentrations and was performed in duplicate.

The whole experiment was repeated with cytochrome C replaced by bovine serum albumin.

Both proteins were radio-labelled with $^{125}$I by a standard technique in which a solution of the protein was mixed with radioactive iodine and chloramine-T, allowed to react, excess iodine removed by addition of tyrosine and the products separated by means of a gel filtration column.

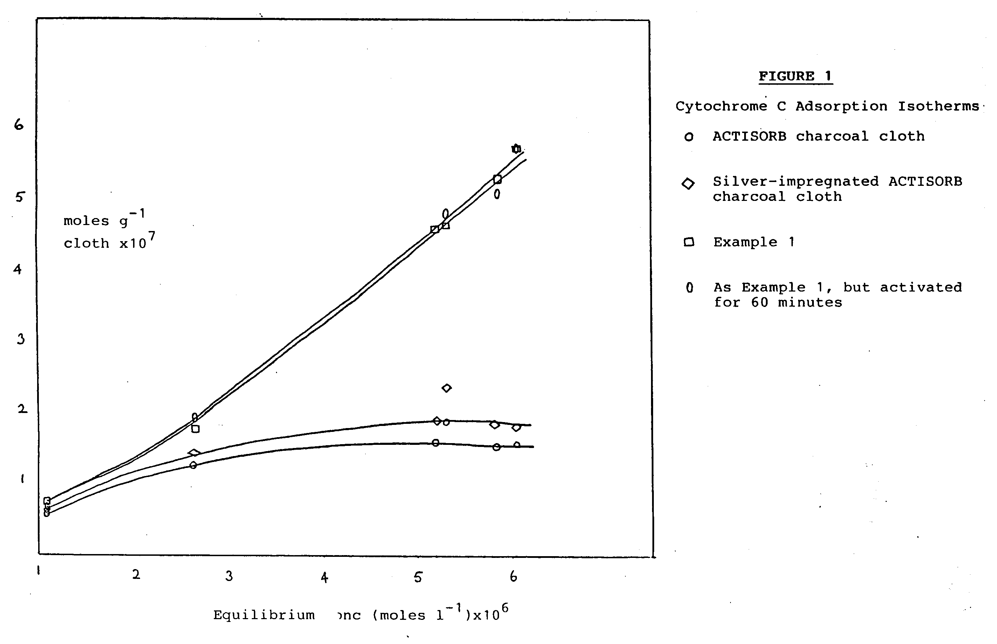

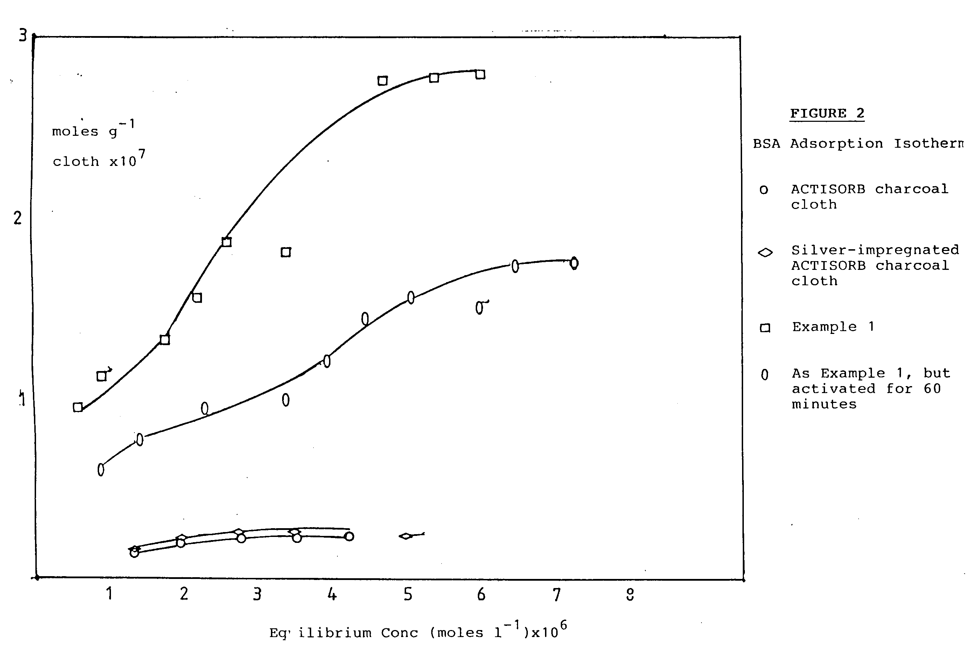

The results were depicted in Figures 1 and 2, along with the results of two control experiments using microporous carbon cloth, prepared in accordance with British Patent No. 1301101. In the first of these control experiments, the carbon cloth used was that which has been used commercially in wound dressings under the Trade Mark ACTISORB. In the second experiment, the carbon cloth is impregnated with a small amount of silver.

As can be seen from Figures 1 and 2, the mesoporous carbon cloths according to the invention are capable of adsorbing far larger quantities of proteins than are the prior art microporous cloths. The effect is especially noticeable at higher protein concentrations. such as those which would be expected to occur in wound exudate. In fact, as will readily be appreciated from Figures 1 and 2, the mesoporous carbon cloths adsorb virtually 100% of the protein in solution, even at relatively high initial concentrations of protein.

Example 5

A sample of viscose rayon cloth was impregnated by immersion for 0.5 minutes in an aqueous solution having the following composition:
boric acid 3% w/v

sodium chloride 0.5% w/v
Excess impregnating solution was then removed by means of blotting paper and the fabric was dried in an oven at 55°C. The sample was then carbonised by heating in an atmosphere of nitrogen with a ramp rate of 10°C min$^{-1}$ from room temperature to 850°C. The furnace was then maintained at a temperature of 850°C under an atmosphere of carbon dioxide for 60 minutes to activate the carbonised fabric.

The resulting product was found to have 25% of its pore volume represented by mesopores.

**Claims**

1.  A wound dressing comprising a layer of activated carbon wherein at least 10% of the pore volume of the activated carbon is represented by mesopores, said dressing being sterile and contained within a bacteria-proof envelope.

2.  A wound dressing according to claim 1 wherein the mesopore content is from 25 to 90% of the total pore volume of the activated carbon.

3.  A wound dressing according to claim 1 or claim 2 wherein the activated carbon is in the form of a fibrous web.

4.  A wound dressing according to any preceding claim wherein the activated carbon is impregnated with an antimicrobial agent.

5.  A wound dressing according to claim 4 wherein the antimicrobial agent is silver.

6.  Activated carbon for use in wound therapy, at least 10% of the pore volume of the carbon being represented by mesopores.

7.  A method of making mesoporous activated carbon for use in a wound dressing, comprising impregnating a cellulosic fibrous material with a liquid medium containing phosphate and an alkali metal, then carbonising and activating the impregnated fibrous material by heating in a suitable atmosphere.

8.  A method according to claim 7 wherein the impregnating medium also contains one or more halides of zinc, aluminium, calcium, magnesium or iron.

9.  A method according to claim 8 wherein the impregnating medium also contains an ammonium salt.

**10.** A method according to any of claims 7 to 9 wherein the impregnating medium also contains an antimicrobial agent.

**11.** A method according to claim 10 wherein the antimicrobial agent is silver.

**Patentansprüche**

**1.** Wundverband, eine Aktivkohle-Schicht umfassend, in der mindestens 10 % des Porenvolumens der Aktivkohle durch Mesoporen dargestellt sind, wobei der genannte Verband steril und in einem bakterienfesten Umschlag enthalten ist.

**2.** Wundverband nach Anspruch 1, bei dem der Mesoporen-Gehalt von 25 bis 90 % des Gesamtporenvolumens der Aktivkohle beträgt.

**3.** Wundverband nach Anspruch 1 oder 2, bei dem die Aktivkohle in Form einer Faserbahn vorliegt.

**4.** Wundverband nach einem der vorstehenden Ansprüche, bei dem die Aktivkohle mit einem antimikrobiellen Mittel imprägniert ist.

**5.** Wundverband nach Anspruch 4, bei dem das antimikrobielle Mittel Silber ist.

**6.** Aktivkohle zur Verwendung in der Wundbehandlung, wobei mindestens 10 % des Porenvolumens der Kohle durch Mesoporen dargestellt sind.

**7.** Verfahren zur Herstellung von Aktivkohle mit Mesoporen zur Verwendung in einem Wundverband, umfassend das Imprägnieren eines Cellulose-Fasermaterials mit einem flüssigen Medium, das Phosphat und ein Alkalimetall enthält, und anschließend das Carbonisieren und Aktivieren des imprägnierten Fasermaterials durch Erhitzen in einer geeigneten Atmosphäre.

**8.** Verfahren nach Anspruch 7, bei dem das Imprägniermedium auch ein oder mehrere Zink-, Aluminium-, Calcium-, Magnesium- oder Eisen-Halogenid(e) enthält.

**9.** Verfahren nach Anspruch 8, bei dem das Imprägniermedium auch ein Anmoniumsalz enthält.

**10.** Verfahren nach einem der Ansprüche 7 bis 9, bei dem das Imprägniermedium auch ein antimikrobielles Mittel enthält.

**11.** Verfahren nach Anspruch 10, bei dem das antimikrobielle Mittel Silber ist.

**Revendications**

**1.** Un pansement pour les blessures comprenant une couche de charbon actif dans lequel au moins 10% du volume de pore du charbon actif est représenté par des mésopores, ledit pansement étant stérile et contenu dans une enveloppe imperméable aux bactéries.

**2.** Un pansement pour les blessures selon la revendication 1, dans lequel la teneur en mésopores représente de 25 à 90% du volume de pore total du charbon actif.

**3.** Un pansement pour les blessures selon la revendication 1 ou la revendication 2, dans lequel le charbon actif est sous la forme d'un tissu fibreux.

**4.** Un pansement pour les blessures selon l'une des revendications précédentes, dans lequel le charbon actif est imprégné d'un agent antimicrobien.

**5.** Un pansement pour les blessures selon la revendication 4, dans lequel l'agent anti-microbien est de l'argent.

**6.** Charbon actif destiné à être utilisé pour la thérapie des blessures, au moins 10% du volume de pore du charbon étant représenté par des mésopores.

**7.** Un procédé de fabrication de charbon actif mésoporeux destiné à être utilisé dans un pansement pour les blessures, comprenant les étapes consistant à imprégner un matériau fibreux cellulosique avec un milieu liquide contenant du phosphate et un métal alcalin, puis de carboniser et à activer le matériau fibreux imprégné en le chauffant dans une atmosphère appropriée.

**8.** Un procédé selon la revendication 7, dans lequel le milieu imprégnant contient un ou plusieurs halogénures de zinc, d'aluminium, de calcium, de magnésium ou de fer.

**9.** Un procédé selon la revendication 8, dans lequel le milieu imprégnant contient également un sel d'ammonium.

**10.** Un procédé selon l'une des revendications 7 à 9, dans lequel le milieu imprégnant contient également un agent anti-microbien.

**11.** Un procédé selon la revendication 10, dans lequel l'agent anti-microbien est de l'argent.

FIGURE 1

Cytochrome C Adsorption Isotherms

O    ACTISORB charcoal cloth

◇    Silver-impregnated ACTISORB
     charcoal cloth

▢    Example 1

0    As Example 1, but activated
     for 60 minutes

EP 0 311 364 B1

FIGURE 2

BSA Adsorption Isotherm

o    ACTISORB charcoal
     cloth

◇    Silver-impregnated
     ACTISORB charcoal
     cloth

□    Example 1

0    As Example 1, but
     activated for 60
     minutes